(19) Europäisches Patentamt
European Patent Office
Office européen des brevets

(11) **EP 3 474 290 B1**

(12) **EUROPEAN PATENT SPECIFICATION**

(45) Date of publication and mention
of the grant of the patent:
**19.06.2024 Bulletin 2024/25**

(21) Application number: **18200343.4**

(22) Date of filing: **15.10.2018**

(51) International Patent Classification (IPC):
**G16H 50/50** (2018.01)  **A61B 5/11** (2006.01)

(52) Cooperative Patent Classification (CPC):
**G16H 50/50; A61B 5/11**

(54) **SYSTEMS AND METHODS FOR OPTIMIZING A JOINT COST FUNCTION AND DETECTING NEURO MUSCULAR PROFILES THEREOF**

SYSTEME UND VERFAHREN ZUR OPTIMIERUNG EINER GEMEINSAMEN KOSTENFUNKTION UND ZUM ERFASSEN VON NEURO-MUSKULÄREN PROFILEN DAVON

SYSTÈMES ET PROCÉDÉS POUR OPTIMISER UNE FONCTION DE COÛT CONJOINT ET DÉTECTER DES PROFILS NEURO MUSCULAIRES ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.10.2017 IN 201721037061**

(43) Date of publication of application:
**24.04.2019 Bulletin 2019/17**

(73) Proprietor: **Tata Consultancy Services Limited Maharashtra (IN)**

(72) Inventors:
• **PARAKKAL UNNI, Midhun**
**560066 Bangalore, Karnataka (IN)**
• **SINHA, Aniruddha**
**700160 Kolkata, West Bengal (IN)**
• **CHAKRAVARTY, Kingshuk**
**560066 Bangalore, Karnataka (IN)**
• **CHATTERJEE, Debatri**
**700160 Kolkata, West Bengal (IN)**
• **DAS, Abhijit**
**700082 Kolkatta, West Bengal (IN)**

(74) Representative: **Goddar, Heinz J.
Boehmert & Boehmert
Anwaltspartnerschaft mbB
Pettenkoferstrasse 22
80336 München (DE)**

(56) References cited:
US-A1- 2004 021 660    US-A1- 2008 031 512
US-A1- 2011 054 870    US-A1- 2015 094 564
US-A1- 2017 287 146

**Description**

TECHNICAL FIELD

The disclosure herein generally relate to sensory motor profiles detection techniques and systems, and, more particularly, to systems and methods for optimizing a joint cost function and detecting neuro muscular profiles thereof.

BACKGROUND

Arguably the most important purpose of the brain is to sense and control movement. A clear understanding of the science behind sensory motor control is still out of reach. A simple task such as flexion of the arm is the result of a highly evolved sophisticated system involving both proprioception and motor control. While a normal subject performs this task effortlessly, patients with sensory-motor disabilities find it difficult to make many of these movements and hence early detection of these disorders is very hard in a normal clinical setting(s).

Document US 2017287146 discloses an apparatus, system and process for tracking and analyzing target person movements, captured while the target person is performing ordinary tasks outside of a medical context, for medical diagnosis and treatment review are described. The method may include constructing a model of a target person from three-dimensional (3D) image data of the target person performing an activity over a period of time. The method may also include tracking movement of the model of the target person in the 3D image data over the period of time, and detecting one or more motion features in the movement of the model of the target person that are relevant to diagnosis, treatment, care, or a combination thereof, of a potential chronic neurodegenerative or musculoskeletal medical condition. The method may also include computing a risk score associated with likelihood of the target person having the medical condition based on the detected motion features.

Document US 2011054870 discloses a system, method, and computer program product for providing a user with a virtual environment in which the user can perform guided activities and receive feedback are described. The user is provided with guidance to perform certain movements. The user's movements are captured in an image stream. The image stream is analyzed to estimate the user's movements, which is tracked by a user-specific human model. Biomechanical quantities such as center of pressure and muscle forces are calculated based on the tracked movements. Feedback such as the biomechanical quantities and differences between the guided movements and the captured actual movements are provided to the user.

SUMMARY

Embodiments of the present disclosure present technological improvements as solutions to one or more of the above-mentioned technical problems recognized by the inventors in conventional systems. The first embodiment concerns a processor implemented method for optimizing a joint cost function and detecting one or more neuro muscular profiles thereof, as defined in appended claim 1. The method comprising obtaining from a Kinect® sensor, neuro motor data pertaining to a User Under Observation (UUO), wherein the neuro motor data comprises information pertaining to a plurality of skeletal joints specific to one or more actions being performed by the UUO; scaling a joint model by identifying the one or more actions performed on the plurality of skeletal joints, wherein the step of scaling a joint model is based on the one or more actions that are extracted by tracking real world motion of the UUO; parameterizing the scaled joint model using one or more physiologically interpretable model parameters to obtain a joint cost function (JC), wherein the one or more physiologically interpretable model parameters comprise one or more applied torques at one or more skeletal joints of the plurality of skeletal joints in the joint model, velocity of motion at the one or more skeletal joints under consideration and one or more Euler angles of the one or more skeletal joints. The method further comprises optimizing, using an inverse optimal control technique, the joint cost function by tuning the one or more physiologically interpretable model parameters; determining a level of the tuning of the one or more physiologically interpretable model parameters based on the optimized joint cost function; performing based on the level of the tuning, a comparison between the one or more tuned physiologically interpretable model parameters and one or more corresponding model parameters pre-computed for a normal user (NU); and detecting one or more neuro muscular profiles based on the comparison.

The step of detecting one or more neuro muscular profiles comprises determining a variation in one or more values associated with the one or more tuned physiologically interpretable model parameters when compared with the one or more corresponding model parameters pre-computed for the normal user (NU).

In an embodiment, the variation in the one or more values is indicative of (indicates) presence and severity of the one or more neuro muscular profiles, wherein the profiles comprise one or more states of the UUO.

The second embodiment concerns a system for optimizing a joint cost function and detecting one or more neuro muscular profiles thereof as defined in appended claim 3. The system comprising: a memory storing instructions and a database; one or more communication interfaces; and one or more hardware processors coupled to the memory via the one or more communication interfaces, wherein the one or more hardware processors are configured by the instructions to: obtain from a Kinect® sensor, neuro motor data pertaining to a user under observation (UUO), wherein the neuro motor data comprises information pertaining to a plurality of skeletal joints specific to one or more actions being performed by the UUO, scale a joint model by identifying the one or more actions performed on the plurality of skeletal joints, parameterize the scaled joint model using one or more physiologically interpretable model parameters to obtain a joint cost function (JC), optimize, using an inverse optimal control technique, the joint cost function by tuning the one or more physiologically interpretable model parameters, determine a level of the tuning of the one or more physiologically interpretable model parameters based on the optimized joint cost function, perform based on the level of the tuning, a comparison between the one or more tuned physiologically interpretable model parameters and one or more corresponding model parameters pre-computed for a normal user (NU), and detect one or more neuro muscular profiles based on the comparison.

The one or more neuro muscular profiles are detected by determining a variation in one or more values associated with the one or more tuned physiologically interpretable model parameters when compared with the one or more corresponding model parameters pre-computed for the normal user (NU), wherein the variation in the one or more values is indicative of presence and severity of the one or more neuro muscular profiles.

In an embodiment, the joint model is scaled based on the one or more actions that are extracted by tracking real world motion of the UUO.

The one or more physiologically interpretable model parameters comprise one or more applied torques at one or more skeletal joints of the plurality of skeletal joints in the joint model, velocity of motion at the one or more skeletal joints under consideration and one or more Euler angles of the one or more skeletal joints.

The third embodiment concerns one or more non-transitory machine readable information storage mediums comprising one or more instructions as defined in appended claim 5. The one or more instructions which when executed by one or more hardware processors causes optimizing a joint cost function and detecting one or more neuro muscular profiles thereof by obtaining from a Kinect® sensor, neuro motor data pertaining to a user under observation (UUO), wherein the neuro motor data comprises information pertaining to a plurality of skeletal joints specific to one or more actions being performed by the UUO; scaling a joint model by identifying the one or more actions performed on the plurality of skeletal joints, wherein the step of scaling a joint model is based on the one or more actions that are extracted by tracking real world motion of the UUO; parameterizing the scaled joint model using one or more physiologically interpretable model parameters to obtain a joint cost function (JC), wherein the one or more physiologically interpretable model parameters comprise one or more applied torques at one or more skeletal joints of the plurality of skeletal joints in the joint model, velocity of motion at the one or more skeletal joints under consideration and one or more Euler angles of the one or more skeletal joints. The method further comprises optimizing, using an inverse optimal control technique, the joint cost function by tuning the one or more physiologically interpretable model parameters; determining a level of the tuning of the one or more physiologically interpretable model parameters based on the optimized joint cost function; performing based on the level of the tuning, a comparison between the one or more tuned physiologically interpretable model parameters and one or more corresponding model parameters pre-computed for a normal user (NU); and detecting one or more neuro muscular profiles based on the comparison.

The step of detecting one or more neuro muscular profiles comprises determining a variation in one or more values associated with the one or more tuned physiologically interpretable model parameters when compared with the one or more corresponding model parameters pre-computed for the normal user (NU).

In an embodiment, the variation in the one or more values is indicative of (indicates) presence and severity of the one or more neuro muscular profiles, wherein the profiles comprise one or more states of the UUO.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for optimizing a joint cost function and detecting neuro muscular profiles thereof according to an embodiment of the present disclosure.

FIG. 2 illustrates an exemplary block diagram illustrating a method for optimizing a joint cost function and detecting neuro muscular profiles thereof in accordance with an embodiment of the present disclosure.

FIG. 3 illustrates an exemplary flow diagram of a method for optimizing a joint cost function and detecting neuro muscular profiles thereof using the system of FIG. 1 in accordance with an embodiment of the present disclosure.

FIGS. 4A-4C depict a graphical representation illustrating the scaled joint model predicting torques for wrist, shoulder and elbow joints for 4 normal subject samples in accordance with an example embodiment of the present disclosure.

FIG. 4D depicts a graphical representation illustrating the scaled joint model being personalized to match the experimental observations of displacement in accordance with an example embodiment of the present disclosure.

FIG. 5 depicts a graphical representation illustrating modeled velocity profiles matching qualitatively with the Kinect® data in accordance with an example embodiment of the present disclosure.

FIGS. 6A-6C depict a graphical representation illustrating variation in $\theta_p$ (state penalty) and $\omega_p$ (velocity penalty) with average applied torques in joints for a set of subjects in accordance with an example embodiment of the present disclosure.

FIGS. 7A-7B depict graphical representations illustrating variation in $\theta_p$ (state penalty) and $\omega_p$ (velocity penalty) with respect to average wrist velocities in different subjects in accordance with an example embodiment of the present disclosure.

FIG. 8A-8B depict variation in $\theta_p$ and $\omega_p$ for normal user(s) and user(s) under observation (patient) categories in accordance with an example embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts.

Unimaginable are the difficulties faced by patients affected by sensory-motor disabilities while executing day to day activities. The flamboyant progress that has made in other areas of medical science does not translate itself in diagnosing them. Early detection and personalized therapy of these disorders is still out of reach. The key issue in dealing with this problem is the lack of understanding of the fundamental mechanism of neural control of motion. Hands and dexterity play a key role in our development as a dominant species on earth. The present disclosure, therefore, chose to model the problem of arm flexion. During motion of the arm, a set of torques is invoked by the neuro-mechanical system to obtain a trajectory. One way to determine these torques is to assume a priori that human body optimizes a particular functional in selecting the right trajectory. This can be anything from total energy, jerk to variance.

However, there is no assumption made that the neural control system is optimizing a readily interpretable functional with predefined physical meaning. Instead, the embodiments of the present disclosure intend to find this cost functional such that the trajectory of motion is optimal and interpret its physical meaning later. This helps in quantifying the neural process as it is, without making any rigid assumptions about nature of the joint cost functional. Here the present disclosure provides a semi-analytic-inverse optimal control method to solve this problem. The methodology used here estimates a set of parameters which are physiologically interpretable. This helps in personalized therapy of the patients and early detection of sensory-motor disorders.

The present disclosure implements a system that assumes that there exist a functional which is minimized by the neuro-mechanical control system and this functional is parameterized by applied torques, the velocity of motion and the Euler angles of the joints. Although there can be a subjective variability in the cost functional being minimized, the structure of it is assumed to remain fixed. The present disclosure believes that finding the right functional that is being minimized in a subject helps in understanding their neuro-mechanical priorities. This problem belongs to the class of bi-level optimization, where an optimized solution is sought at two levels. One at the level of finding the right trajectory (state as a function of time) and the other at finding the right cost functional given the trajectory is optimal.

In the present disclosure, the solution for the first problem is obtained by using algebraic Riccati formulation and the second optimization problem is solved using downhill simplex algorithm. The present disclosure implements Microsoft Kinect® for the data collection as it is a low-cost device with satisfactory accuracy. This system is increasingly being used in the rehabilitation and physical therapy. Furthermore, these disorders are detected once the condition reaches its full-blown state is trivial.

A model developed to study these disorders should be able to delineate the mild variations in the neuro-motor function. This helps the diagnostician at an early stage to manage the condition effectively. Therefore the ideal set of subjects to be used to validate the methodology are those who have a mild form of these disabilities such as elderly and ones with diabetes mellitus. Hereinafter subjects may be referred as one of patients, abnormal patients, abnormal subjects, user under observation or subject under observation, and the like and may be interchangeably used. Besides detecting sensory-motor disabilities in diabetes, the present disclosure could benefit the patients with motor disorders such as Parkinson's disease and Huntington's.

The embodiments of the present disclosure provide systems and methods for optimizing a joint cost function and detecting neuro muscular profiles thereof. The system implements a model (that is developed) that quantifies these disorders in terms of a cost functional, which captures the trade-off between the torques applied and the velocities experienced at the joints. Estimation of this cost functional, otherwise known as Inverse optimal control was then carried out using an optimization procedure. To validate the ability of this estimated cost functional to distinguish weakly distinct neuro-motor conditions, the systems and methods of the present disclosure used Microsoft Kinect® motion capture data from normal subjects (or referred as normal users hereinafter and may be interchangeably used) and a patient population with mild sensory-motor disabilities due to old age and Diabetes Mellitus.

Movement is central to the all-important biological activities of animals from feeding and fleeing to procreation. Coordinated activities by the brain and spinal cord results in controlled motor patterns. Conditions such as Diabetes and Parkinson's affect this motion control circuitry at some stage of their progression. Determination of muscle activation during a movement is an inverse problem. In its 'naive' form this is ill-posed. There are multiple solutions for muscle activations that can result in the same trajectory of motion. Methods currently in use approach this problem using dynamic or static optimization techniques and validate the results using EMG studies when necessary.

Evolutionarily, the human nervous system is designed to choose the 'right' set of activations for every trajectory of motion. As every search problem has a dual in optimization it can be safely assumed that the human physiological system is trying to optimize a cost functional. Research studies have shown that the sensory information is important to achieve optimal control. It is therefore anticipated that finding the right cost functional would reveal information about both sensory and motor systems of a subject. The embodiments of the present disclosure suggest a methodology to find the cost functional for an individual subject during movement. As the cost functional considers the activity of all joints in motion, it is hereinafter referred as the 'joints cost (JC)' for the trajectory.

Optimal control - As Neuro-mechanical Control Technique

JC for a task varies between subjects and even within a subject over time. Brain and spinal cord minimize this JC throughout the movement. There is plurality of hypotheses about what exactly the nature of this JC is and how it is being minimized. The JC can be a function of metabolic energy, jerk or variance in motion. But it can be readily observed that JC for the system can be approximated to a function of applied torques and states. The problem of finding the right control trajectory and minimizing the JC is an optimal control problem. The present disclosure assumes the existence of a JC parameterized by states which are optimized by the neuro-mechanical control system for every trajectory of motion.

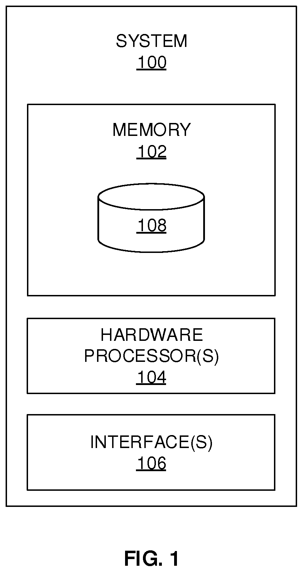

Referring now to the drawings, and more particularly to FIGS. 1 through 8B, where similar reference characters denote corresponding features consistently throughout the figures, there are shown preferred embodiments and these embodiments are described in the context of the following exemplary system and/or method.

FIG. 1 illustrates an exemplary block diagram of a system for optimizing a joint cost function and detecting neuro muscular profiles thereof according to an embodiment of the present disclosure. In an embodiment, the system 100 includes one or more processors 104, communication interface device(s) or input/output (I/O) interface(s) 106, and one or more data storage devices or memory 102 operatively coupled to the one or more processors 104. The one or more processors 104 may be one or more software processing modules and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the device 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment a database 108 can be stored in the memory 102, wherein the database 108 may comprise, but are not limited to information pertaining to one or more user(s) under observation (UUO), normal user data, one or more physiologically interpretable model parameters pertaining to the UUO, a joint cost function (JC) of users, tuning information of the above parameters, one or more neuro muscular profiles (e.g., state of the UUO) being detected based on the JC being optimized and the like). In an embodiment, the memory 102 may store one or more joint models (not shown in FIG. 1) and the like, which are scaled/personalized by the one or more hardware processors 104 (or by the system 100) to perform the methodology described herein.

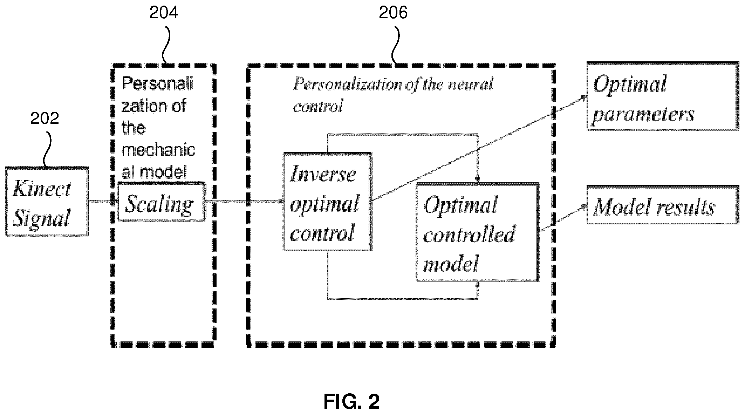

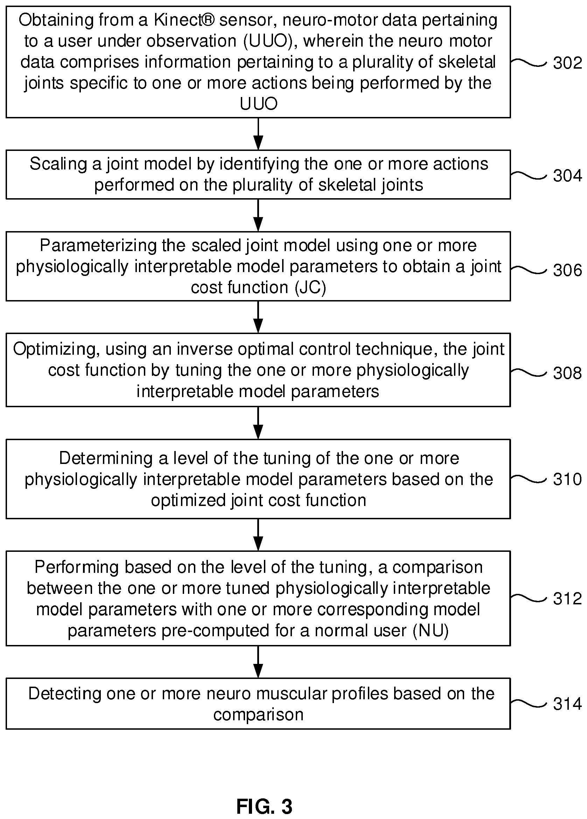

FIG. 2, with reference to FIG. 1, illustrates an exemplary block diagram illustrating a method for optimizing a joint cost function and detecting neuro muscular profiles thereof in accordance with an embodiment of the present disclosure. FIG. 3, with reference to FIGS. 1-2, illustrates an exemplary flow diagram of a method for optimizing a joint cost function and detecting neuro muscular profiles thereof using the system 100 of FIG. 1 in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to the components of the system 100 as depicted in FIG. 1, and FIG. 2, and the flow diagram of FIG. 3. In an embodiment of the present disclosure, at step 302, the one or more hardware processors 104 obtain from a Kinect® sensor, neuro-motor data (or Kinect® signal) pertaining to a user under observation (UUO), wherein the neuro motor data comprises information pertaining to a plurality of skeletal joints specific to one or more actions being performed by the UUO. In an embodiment of the present disclosure, the input (e.g., neuro-motor data or Kinect® signal) that is fed to the system 100 is as shown in block 202 of FIG. 2. In an example embodiment, Kinect® data after re-sampling (30 fps to 60 fps - wherein fps refers to frames per second) was used as the input to the system 100. In an embodiment of the present disclosure, at step 304, the one or more hardware processors 104 scale (or personalize) a joint model (also referred as 'neuro motor model') by identifying the one or more actions performed on the plurality of skeletal joints. More particularly, block 204 depicts the scaling of joint model in FIG. 2, in an example embodiment. In an example embodiment, after re-sampling the Kinect® data, it was then scaled to correct the joint model with the joint coordinates of corresponding subject (e.g., the UUO). In an embodiment of the present disclosure, the step of scaling a joint model is based on the actions performed by the one or more skeletal joints that are extracted by tracking real world motion of the UUO. In an embodiment, joint model is a depiction of the dynamic behavior of one or more skeletal joint(s) from the plurality of joints.

In an embodiment of the present disclosure, at step 306, the one or more hardware processors 104 parameterize the scaled joint model using one or more physiologically interpretable model parameters to obtain a joint cost function (JC). In an embodiment of the present disclosure, joint cost function (JC) refers to cost (e.g., energy, smoothness, and the like) associated with movement of one or more skeletal joint(s) against the gravity to perform one or more task(s). In an embodiment, the one or more physiologically interpretable model parameters comprise, but are not limited to, one or more applied torques at one or more skeletal joints of the plurality of skeletal joints in the joint model, velocity of motion at the one or more skeletal joints under consideration and/or one or more Euler angles of the one or more skeletal joints from the plurality of joints. In an embodiment of the present disclosure, at step 308, the one or more hardware processor 104 optimize, using an inverse optimal control technique, the joint cost function by tuning the one or more physiologically interpretable model parameters. In an embodiment, post scaling of the joint model, the output is then fed to an inverse optimal control technique (see block 206 of FIG. 2) where the functional J is optimized to match the Kinect® data. The optimal control in itself involved an optimization of a functional using variational calculus formulations where the search is done in space of smooth functions matching the boundary conditions. In an embodiment of the present disclosure, at step 310, the one or more hardware processors 104 determine a level of the tuning of the one or more physiologically interpretable model parameters based on the optimized joint cost function.

In an embodiment of the present disclosure, at step 312, the one or more hardware processors 104 perform based on the level of the tuning, a comparison between the one or more tuned physiologically interpretable model parameters with one or more corresponding model parameters pre-computed (or pre-defined) for a normal user (NU). In an embodiment, the one or more corresponding model parameters pre-computed for the normal user (NU) are stored in the database 108 and retrieved by query the database 108 for comparison. In an embodiment of the present disclosure, the one or more tuned physiologically interpretable model parameters are tuned to generate or obtained optimal tuned parameters for the UUO. In an embodiment of the present disclosure, at step 314, the one or more hardware processors 104 detect one or more neuro muscular profiles based on the comparison. In an embodiment, the one or more neuro muscular profiles comprise but are not limited to, one or more states (e.g., normal, abnormal, and the like) of the UUO. In an embodiment, the one or more neuro muscular profiles are detected when variation(s) in one or more values associated with the one or more tuned physiologically interpretable model parameters are determined in comparison to the one or more corresponding model parameters pre-computed for the normal user (NU), wherein the variation(s) in the one or more values is indicative of presence and severity of the one or more neuro muscular profiles.

Below illustrated is an existing approach and the proposed methodology:
Movement is central to the all-important biological activities of animals from feeding and fleeing to procreation. Coordinated activities by the brain and spinal cord results in controlled motor patterns. Conditions such as Diabetes and Parkinson's affect this motion control circuitry at some stage of their progression. Determination of muscle activation during a movement is an inverse problem. In its 'naïve' form this is ill-posed. There are multiple solutions for muscle activations that can result in the same trajectory of motion. Methods currently in use approach this problem using dynamic or static optimization techniques and validate the results using EMG studies when necessary.

Evolutionarily, the human nervous system is designed to choose the 'right' set of activations for every trajectory of motion. As every search problem has a dual in optimization it can be safely assumed that the human physiological system is trying to optimize a cost functional. Research studies have shown that the sensory information is important to achieve optimal control. It is therefore anticipated that finding the right cost functional would reveal information about both sensory and motor systems of a subject. The embodiments of the present disclosure suggest a methodology to find the cost functional for an individual subject during movement. As the cost functional considers the activity of all joints in motion, it is hereinafter referred as the 'joints cost (JC)' for the trajectory.

**Optimal control - As Neuro-mechanical Control Technique**

JC for a task varies between subjects and even within a subject over time. Brain and spinal cord minimize this JC throughout the movement. There is plurality of hypotheses about what exactly the nature of this JC is and how it is being minimized. The JC can be a function of metabolic energy, jerk or variance in motion. But it can be readily observed that JC for the system can be approximated to a function of applied torques and states. The problem of finding the right control trajectory and minimizing the JC is an optimal control problem. The present disclosure assumes the existence of a JC parameterized by states which are optimized by the neuro-mechanical control system for every trajectory of motion.

**Optimal Control-Formalism:**

An optimization problem of a functional with a differential constraints can be solved in the following way as illustrated by existing or conventional research studies/methodologies. Let the functional to be minimized be:

$$J = \int_0^\infty (\boldsymbol{X}^T Z \boldsymbol{X} + c^T U c) dt \quad (1)$$

and the differential constraint be

$$\frac{d\boldsymbol{X}}{dt} = A\boldsymbol{X}(t) + Bc(t) \quad (2)$$

and $\boldsymbol{X}(t_0) = \boldsymbol{X}0$
then the optimal

$$c(t) = -K\boldsymbol{X}(t) \quad (3)$$

where

$$-SA - A^T S + SBU^{-1}B^T S - Z = 0 \quad (5)$$

Proposed approach by the embodiments of the present disclosure and the system:

**Mathematical model of the Arm - The equations of motion**

The proposed disclosure developed a planar 3 linked arm model with revolute joints. A sigmoid nonlinear constraint function ($k \in C^\infty$)[1] was imposed on the joints to avoid elbow hyperextension. The links were assumed to be rigid bodies with constant inertial properties. The reference frame was chosen to be shoulder joint and was considered to be at rest. The torques acting on different joints were modeled to be a function of time which were controlled by a control parameter c. Gravity was assumed to be acting at the center of mass of the links. The nonlinear constraint function was assumed to be a sigmoid function for its smoothness and ability to achieve arbitrary levels of accuracy. Kanes method was employed to derive the expressions of motion given as:

$$f + f^* = 0 \quad (6)$$

Where $f$ is the generalized active force vector and $f^*$ is the generalized inertial force vector. These set of equations were then converted and assembled into the following form and solved as expressed below by way of example:

$$M\frac{d\boldsymbol{X}}{dt} = r \quad (7)$$

Where $M$ is the mass matrix and $\boldsymbol{X}$ the state vector and $r$ is the forcing vector. Here the generalized coordinates and speeds were chosen to be angles and angular velocities respectively.

**Optimal control as neural control**

The system of expression (or equation) 7 is nonlinear. This was linearized at an unstable equilibrium point to convert to the following expression below illustrated by way of example:

$$\frac{d\boldsymbol{X}}{dt} = A\boldsymbol{X} + Bc \quad (8)$$

Where $\boldsymbol{A} \in \mathbb{C}^{6\times6}$ and $\boldsymbol{B} \in \mathbb{C}^{6\times3}$ represents the linearized coefficients of the differential equation. $C^n$ is the set of all n times differentiable functions, and $\mathbb{C}^{m\times n}$ is the set of all $m \times n$ matrices. The model was scaled for each UUO (or subject) to match the experimentally obtained lengths of the different joints. Expression/equation (9) was then used as the cost functional. First term inside the integral represents cost on changes in state $\boldsymbol{X}$ and the second term represents the cost on controls c. The final time of the integral is assumed $\infty$ as the UUO were not given an end time to complete the task.

$$J = \int_0^\infty (\boldsymbol{X}^T Z \boldsymbol{X} + c^T U c)\,dt \quad (9)$$

$$\boldsymbol{X} = [\theta 1, \theta 2, \theta 3, \omega 1, \omega 2, \omega 3] \quad (10)$$

Where $\theta(1$ - $3)$s are the joint angles in should, elbow and wrist respectively and $\omega(1$ - $3)$s are the angular velocities. $\boldsymbol{X}$ is the state variable, c the control torques and $Z \in \mathbb{C}^{6\times6}$ and $U \in \mathbb{C}^{3\times3}$ are the weights of independent and controllable variables of the differential equation respectively. These were constrained to be positive definite. As the function was linearized at the final state $\boldsymbol{X}^*$ and minimization of the cost-function $J$ results in minimization of the Euclidean distance between this equilibrium point and the current state. Here the matrices $Z$ and $U$ were assumed to be diagonal and arbitrary with the following structure for $Z$.

$$Z = \begin{bmatrix} \theta_p & 0 & 0 & 0 & 0 & 0 \\ 0 & \theta_p & 0 & 0 & 0 & 0 \\ 0 & 0 & \theta_p & 0 & 0 & 0 \\ 0 & 0 & 0 & \omega_p & 0 & 0 \\ 0 & 0 & 0 & 0 & \omega_p & 0 \\ 0 & 0 & 0 & 0 & 0 & \omega_p \end{bmatrix} \quad (11)$$

Where $\theta_p$ and $\omega_p$ represent penalty to angular displacement and velocities respectively. The embodiments of the present disclosure assumed $U = \mathbb{I}^{3\times3}$ the identity matrix. In the inverse optimal control problem (as depicted in FIG. 2) a requirement for increased penalty to torques applied would translate to an appropriate change in the penalties $\theta_p$ and $\omega_p$. Making a set of weights constant in relation to another also helps in reducing the search space for the algorithm/technique thus reducing the computational cost considerably. The problem was formulated as an infinite horizon to account the fact that final time was not given to the patients as a criterion while performing the experiments. In summary the cost functional $J$ was solved with differential constraints in Equation (8). The inverse optimal control problem of determining the matrix $Z$ was done using a 'simplex method for function minimization technique', and/or 'direct search method(s)' known in the art. The overall algorithm/technique is depicted in FIG. 2. Mathematically the optimization problem of finding the personalized $J^*$ can be described as in the following way. Each $J$ maps to only one trajectory x; this makes x function of $J$ represented from hereon as x($J$)

$$J^* = \underset{J \in c^2}{argmin}\, \boldsymbol{Er}(\mathrm{x}(\boldsymbol{J}), \tilde{\mathrm{x}}) \qquad (12)$$

Where x is the solution of the optimal control problem for given $J$ and $\tilde{\mathrm{x}}$ is the measured state trajectory. The function $Er(x(J), \tilde{x})$ maps the trajectories of the states x and $\tilde{x}$ to the $l_2$ norm of the difference between the corresponding displacements in the Cartesian y-coordinate of the wrist. The projection of the data in the way describes helps to reduce the complexity of the optimization problem significantly in a way that is physically meaningful. The data collection is described in below section.

**Data collection using Kinect® and signal processing:**

The range of motion for left shoulder flexion was collected using Microsoft Kinect® XBox One. A total of 13 normal subjects (Male=6, Female=7) and 19 patients (Male=7, Female=12) were selected for the study. The mean (standard deviation) of the age for normal subjects and patients are 30.2 (8.4) years and 65.64 (4.62) respectively. The range of the age for normal subjects and patients are 22 to 45 years and 56 to 74 years respectively. Each session was repeated two times for all normal subjects resulting in a total of 26 samples whereas a single trial was used for patients generating total 19 samples.

The collected data was then resampled to 60 fps from 30 fps for the analysis using built-in python Fourier method. The lengths of different bone segments were then computed for each subject using the 3D coordinates of the skeleton data of the initial 5 seconds of a static window before the start of the exercise. This was used for scaling and the scaled model was given as input to the inverse optimal control algorithm to optimize the cost functional.

**Results:**

The first part of results verify the model by comparing it with the experimentally observed motion and the second part validates the model by finding its effectiveness in differentiating normals from patients with mild sensory-motor disabilities.

**Comparison of model experiments for torque, velocity, and displacements:**

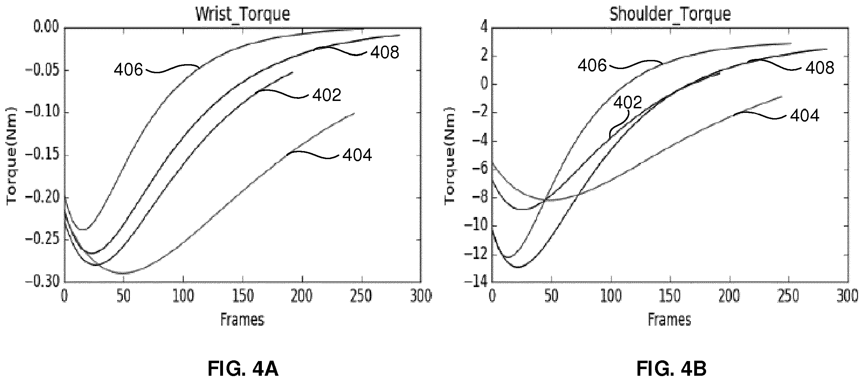

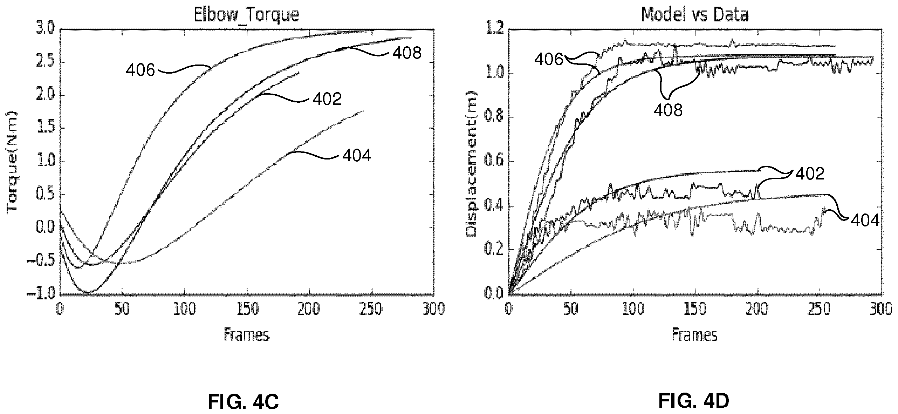

It was observed that the displacements obtained from the model matched with the experimental results from the Kinect® with negligible error (see FIGS. 4A-4B). In the two samples shown represented by 402 and 404, subjects were asked to lift the arms close to 90 degrees and in the other two (406 and 408), subjects were asked to lift the arm to 180 degrees. This tested the ability of the model to match the range of motion. The personalized cost functions for the set of individuals were estimated using the Kinect® marker data using the Equations (8) and (9) respectively. More particularly, FIGS. 4A-4C, with reference to FIGS. 1 through 3, depict a graphical representation illustrating the scaled joint model predicting torques for wrist, shoulder and elbow joints for 4 normal subject samples, and FIG. 4D, with reference to FIGS. 1 through 4C, depicts a graphical representation illustrating the scaled joint model being personalized to match the experimental observations of displacement in accordance with an example embodiment of the present disclosure. The range of motion is very well matched by the model used.

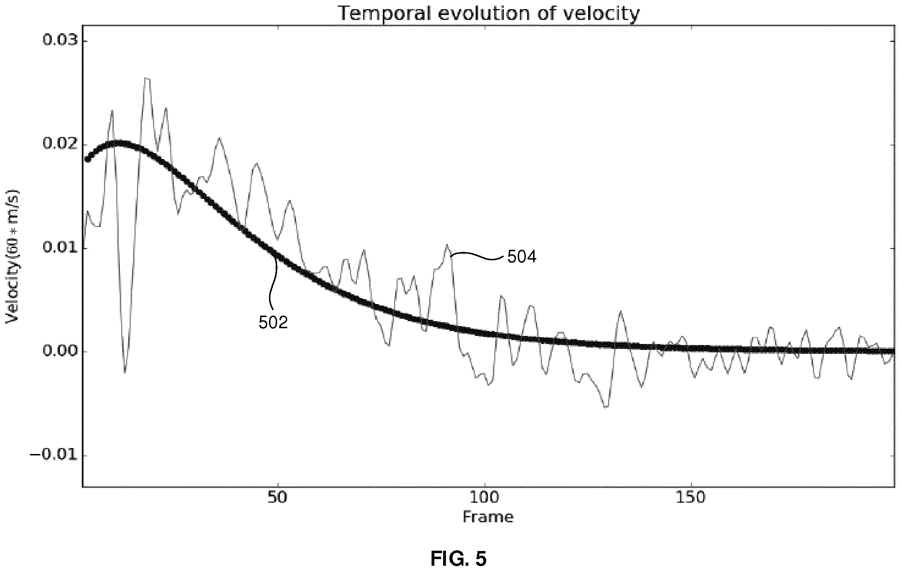

The torques of elbows and wrist were determined using equation (9) and are plotted in FIGS. 4A-4D. It was observed that the values of the torques generated decreased initially and then increased to match the inertial forces. It was also found the velocity of the arm obtained from the scaled joint model followed a nonlinear trajectory similar to observed in experiment (see FIG. 5). More particularly, FIG. 5, with reference to FIGS. 1 through 4D, depicts a graphical representation illustrating modeled velocity profiles matching qualitatively with the Kinect® data in accordance with an example embodiment of the present disclosure. The deviation of the Kinect® data from the scaled joint model is due to the fact that the process of finding the instantaneous velocity involves taking numerical derivatives which amplify the high-frequency noise. Modeled velocity is represented by 502, and the data is shown represented by 504 in FIG. 5.

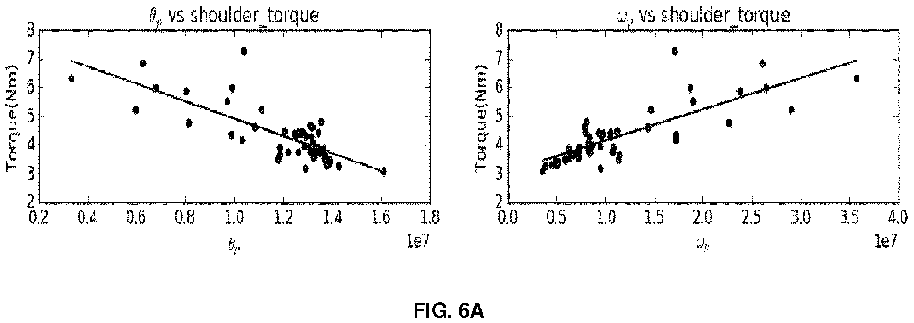

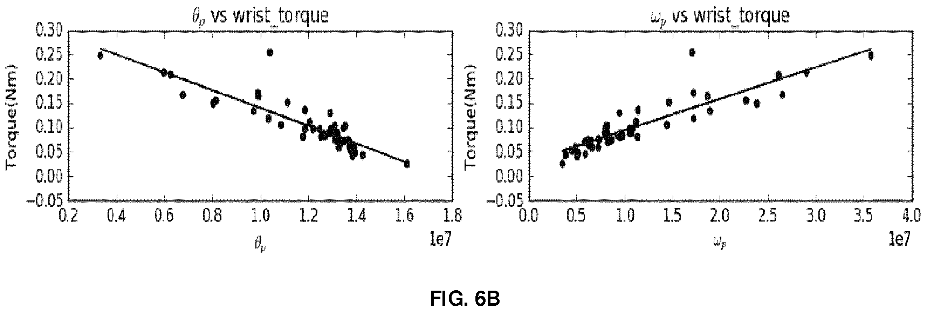

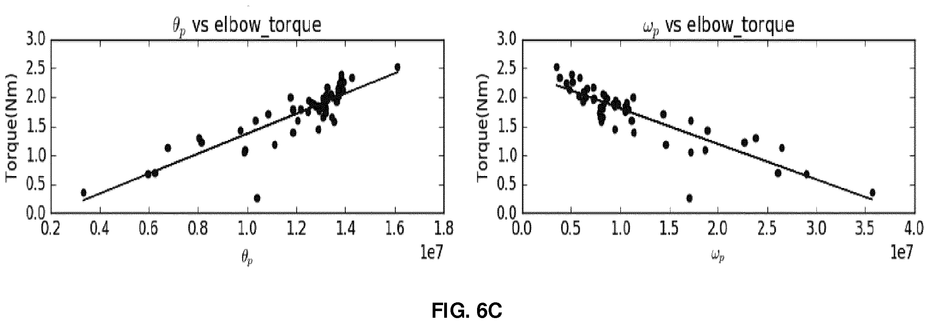

It was further analysed by the system 100 and the embodiments of the present disclosure how the values of parameters $\theta_p$ (state penalty) and $\omega_p$ (velocity penalty) govern the torque values in different subjects for multiple joints. The average torque values for shoulder and wrist joints increased with increase in $\omega_p$ but decreased for elbow joints. An increase in $\theta_p$ is associated with a decrease in shoulder and wrist torques and an increase for elbow. The trade-off between the velocity of motion and torques applied is balanced by the JC results in this diametrical trend. FIG. 6A-6C, with reference to FIGS. 1 through 5, depicts a graphical representation illustrating variation in $\theta_p$ (state penalty) and $\omega_p$ (velocity penalty) with average applied torques in joints for a set of subjects in accordance with an example embodiment of the present disclosure. Each point (represented by black solid dot) in the plot represents a subject.

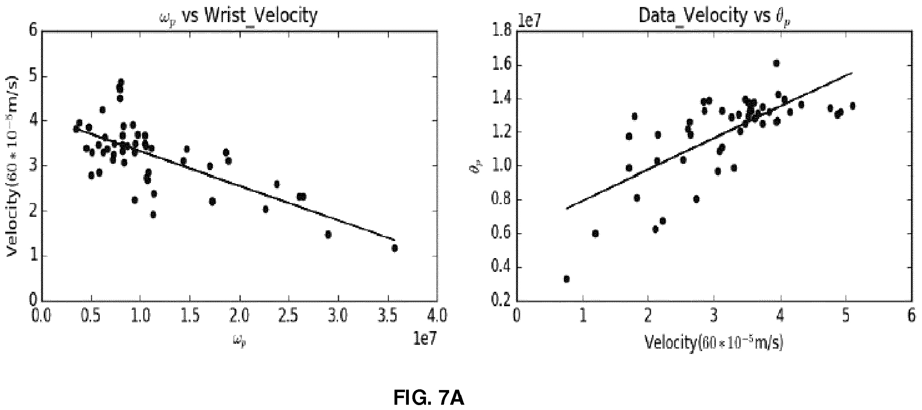

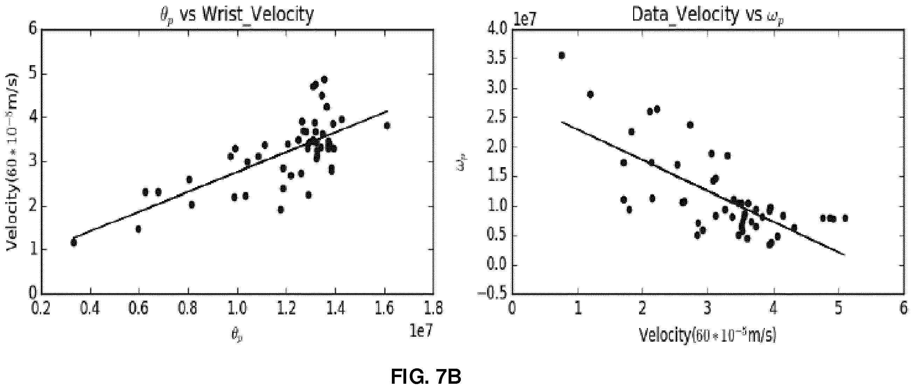

Given the above results, the question remains is it possible to estimate these values without resorting to optimization? The velocity values were fitted with $\theta_p$ and as well as $\omega_p$ using linear regression. It was found that a linear relationship (see FIGS. 7A-7B) between average velocity of the data collected with $\theta_p$ and $\omega_p$. Although very crude, it is believed this trend can be used in setting where the computational power is low. FIGS. 7A-7B, with reference to FIGS. 1 through 6C, depict graphical representations illustrating variation in $\theta_p$ (state penalty) and $\omega_p$ (velocity penalty) with respect to average wrist velocities in different subjects in accordance with an example embodiment of the present disclosure. Each point (represented by black solid dot) in the plot represents a subject.

**Comparison of $\theta_p$ and $\omega_p$ in normal subjects and abnormal subjects (e.g., patients or user(s) under observation):**

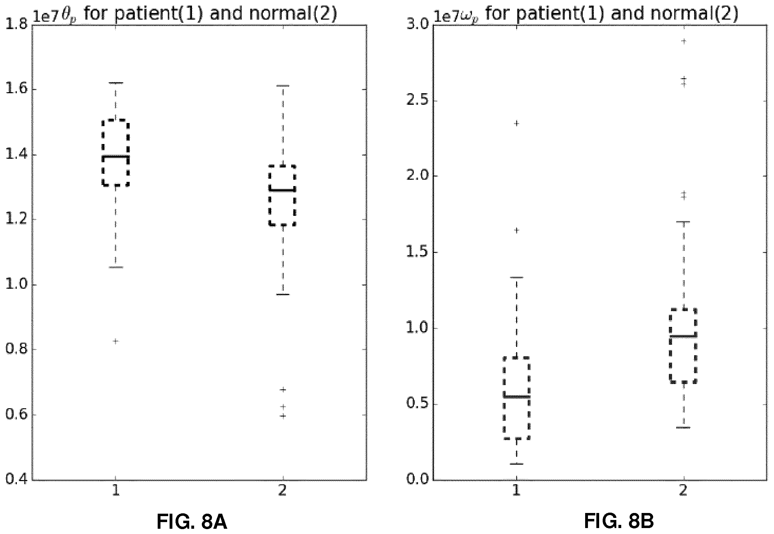

FIG. 8A-8B, with reference to FIGS. 1 through 7B, depict variation in $\theta_p$ and $\omega_p$ for normal user(s) and user(s) under observation (patient) categories in accordance with an example embodiment of the present disclosure. The $\theta_p$ in FIG. 8A is significantly higher in patients (UUO) than in normal (normal users) whereas $\omega_p$ (in FIG. 8B) is significantly lower in patients than in normal subjects (or normal users). More particularly, FIG. 8A-8B show the comparison of the $\theta_p$ and $\omega_p$ in normal user and user under observation (e.g., patient) categories. To analyze the significance a welch's t-test was performed without assuming equal variance or equal number of samples. It was found that these cost function parameters vary significantly between normal users and UUO (see FIGS. 8A-8B) with a test statistic value of -2.28, pvalue 0.027 and test statistic value 2.3, p-value of 0.023 for $\theta_p$ and $\omega_p$ respectively. The test used did not assume the same variance, which if assumed the p-value may be lower. Therefore these parameters can be considered as a good measure to understand the underlying physiology of a patient. A lower $\omega_p$ was observed in patients indicating jerky and fast movements. On the other hand a higher $\theta_p$ change was observed in patients indicating a lower ability to hold the hand during an intermediate position. The objective of the embodiments of the present disclosure was to develop a methodology to quantify and understand the neuro-mechanical pathologies. It was found through the above experimental results and graphical representations as depicted in figures that the inverse optimal control methodology can be used to personalize model of the arm dynamics and joint cost (JC) functional. Thus the JC parameterized by $\theta_p$ and $\omega_p$ can be used to understand subject specific variations in different pathologies.

The estimated cost functional parameterized by state penalty $\theta_p$ and $\omega_p$ velocity penalty is significantly different between normal subjects and patients (p<0.05). A lower $\omega_p$ is observed in patients leading to jerky fast movements and a higher $\theta_p$ indicating a lessened ability to hold the hand in an intermediate position. The above results suggest that cost functional associated with a subject is a good measure to personalize and quantify the underlying movement dynamics.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, BLU-RAYs, DVDs, flash drives, disks, and any other known physical storage media.

## Claims

1. A processor implemented method, comprising:

   obtaining from a Kinect® sensor, neuro motor data pertaining to a User Under Observation (UUO), wherein the neuro motor data comprises information pertaining to a plurality of skeletal joints specific to one or more actions being performed by the UUO (302);
   scaling a joint model for each UUO by identifying the one or more actions performed on the plurality of skeletal joints (304) to match one or more experimentally obtained lengths of the plurality of skeletal joints;
   parameterizing the scaled joint model using one or more physiologically interpretable model parameters to obtain a joint cost function (JC) (306);
   optimizing, using an inverse optimal control technique, the joint cost function, by tuning the one or more physiologically interpretable model parameters (308), wherein the inverse optimal control technique is bi-level optimization in nature and an optimal solution is required at two levels, and wherein a first level requires finding a right trajectory by using algebraic Riccati formulation and a second level is solved using a downhill simplex algorithm;
   determining a level of the tuning of the one or more physiologically interpretable model parameters based on the optimized joint cost function (310);
   performing a comparison using one or more tuned physiologically interpretable model parameters between the plurality of skeletal joints specific to one or more actions being performed by the UUO (302) and one or more corresponding model parameters pre-computed for a normal user (NU) (312) wherein the one or more physiologically interpretable model parameters comprise one or more applied torques at one or more skeletal joints of the plurality of skeletal joints in the j oint model, velocity of motion at the one or more skeletal joints under consideration and one or more Euler angles of the one or more skeletal joints; and

detecting one or more states of a plurality of neuro muscular profiles, wherein the neuro muscular profiles are detected when variation(s) in one or more values associated with the one or more tuned physiologically interpretable model parameters are determined in comparison to the one or more corresponding model parameters pre-computed for the normal user (NU), , and wherein the neuro muscular profiles are modeled velocity profiles that indicate a plot of a temporal evolution of modeled and observed velocity vs. a frame of Kinect data.

2. The processor implemented method of claim 1, wherein the step of scaling a joint model is based on the one or more actions that are extracted by tracking real world motion of the UUO.

3. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

obtain from a Kinect® sensor, neuro motor data pertaining to a User Under Observation (UUO), wherein the neuro motor data comprises information pertaining to a plurality of skeletal joints specific to one or more actions being performed by the UUO, scale a joint model for each UUO by identifying the one or more actions performed on the plurality of skeletal joints to match one or more experimentally obtained lengths of the plurality of skeletal joints;
parameterize the scaled joint model using one or more physiologically interpretable model parameters to obtain a joint cost function (JC);
optimize, using an inverse optimal control technique, the joint cost function, by tuning the one or more physiologically interpretable model parameters, wherein the inverse optimal control technique is bi-level optimization in nature and an optimal solution is required at two levels, and wherein a first level requires finding a right trajectory by using algebraic Riccati formulation and a second level is solved using a downhill simplex algorithm;
determine a level of the tuning of the one or more physiologically interpretable model parameters based on the optimized joint cost function,
perform a comparison using one or more tuned physiologically interpretable model parameters between the plurality of skeletal joints specific to one or more actions being performed by the UUO (302) and one or more corresponding model parameters pre-computed for a normal user (NU), wherein the one or more physiologically interpretable model parameters comprise one or more applied torques at one or more skeletal joints of the plurality of skeletal joints in the joint model, velocity of motion at the one or more skeletal joints under consideration and one or more Euler angles of the one or more skeletal joints;
detect one or more states of a plurality of neuro muscular profiles, wherein the neuro muscular profiles are detected when variation(s) in one or more values associated with the one or more tuned physiologically interpretable model parameters are determined in comparison to the one or more corresponding model parameters pre-computed for the normal user (NU) , and wherein the neuro muscular profiles are modeled velocity profiles that indicate a plot of a temporal evolution of modeled and observed velocity vs. a frame of Kinect data.

4. The system of claim 3, wherein the joint model is scaled based on the one or more actions that are extracted by tracking real world motion of the UUO.

5. One or more non-transitory machine readable information storage mediums comprising one or more instructions which when executed by one or more hardware processors cause:

obtaining from a Kinect® sensor, neuro motor data pertaining to a User Under Observation (UUO), wherein the neuro motor data comprises information pertaining to a plurality of skeletal joints specific to one or more actions being performed by the UUO;
scaling a joint model for each UUO by identifying the one or more actions performed on the plurality of skeletal joints to match one or more experimentally obtained lengths of the plurality of skeletal joints;
parameterizing the scaled joint model using one or more physiologically interpretable model parameters to obtain a joint cost function (JC);
optimizing, using an inverse optimal control technique, the joint cost function, by tuning the one or more physiologically interpretable model parameters , wherein the inverse optimal control technique is bi-level optimization in nature and an optimal solution is required at two levels, and wherein a first level requires finding a right trajectory by using algebraic Riccati formulation and a second level is solved using a downhill simplex algorithm; determining a level of the tuning of the one or more physiologically interpretable model parameters based on the optimized joint cost function; performing a comparison using the one or more tuned physiologically interpretable model parameters between the plurality of skeletal joints specific to one or more actions being performed by the UUO (302) and one or more corresponding model parameters pre-computed for a normal user (NU), wherein the one or more physiologically interpretable model parameters comprise one or more applied torques at one or more skeletal joints of the plurality of skeletal joints in the joint model, velocity of motion at the one or more skeletal joints under consideration and one or more Euler angles of the one or more skeletal joints; and

detecting one or more states of a plurality of neuro muscular profiles wherein the neuro muscular profiles are detected when variation(s) in one or more values associated with the one or more tuned physiologically interpretable model parameters are determined in comparison to the one or more corresponding model parameters pre-computed for the normal user (NU), and wherein the neuro muscular profiles are modeled velocity profiles that indicate a plot of a temporal evolution of modeled and observed velocity vs. a frame of Kinect data.

6. The one or more non-transitory machine readable information storage mediums of claim 5, wherein the step of scaling a joint model is based on the one or more actions that are extracted by tracking real world motion of the UUO.

**Patentansprüche**

1. Prozessorimplementiertes Verfahren, umfassend:

Erhalten von neuromotorischen Daten von einem Kinect®-Sensor, die sich auf einen Benutzer unter Beobachtung (UUO) beziehen, wobei die neuromotorischen Daten Informationen umfassen, die sich auf eine Mehrzahl von Skelettgelenken beziehen, die für eine oder mehrere Aktionen spezifisch sind, die durch den UUO (302) durchgeführt werden; Skalieren eines Gelenkmodells für jeden UUO durch Identifizieren der einen oder mehreren Aktionen, die an der Mehrzahl von Skelettgelenken (304) durchgeführt werden, um mit einer oder mehreren experimentell erhaltenen Längen der Mehrzahl von Skelettgelenken übereinzustimmen; Parametrisieren des skalierten Gelenkmodells unter Verwendung eines oder mehrerer physiologisch interpretierbarer Modellparameter, um eine Gelenkkostenfunktion (JC) (306) zu erhalten; Optimieren der Gelenkkostenfunktion unter Verwendung einer inversen optimalen Steuerungstechnik durch Abstimmen des einen oder der mehreren physiologisch interpretierbaren Modellparameter (308), wobei die inverse optimale Steuerungstechnik eine Bilevel-Optimierung in der Natur ist und eine optimale Lösung auf zwei Ebenen erforderlich ist, und wobei eine erste Ebene das Finden einer richtigen Trajektorie unter Verwendung einer algebraischen Riccati-Formulierung erfordert und eine zweite Ebene unter Verwendung eines Downhill-Simplex-Algorithmus gelöst wird; Bestimmen einer Ebene der Abstimmung des einen oder der mehreren physiologisch interpretierbaren Modellparameter basierend auf der optimierten Gelenkkostenfunktion (310); Durchführen eines Vergleichs unter Verwendung eines oder mehrerer abgestimmter physiologisch interpretierbarer Modellparameter zwischen der Mehrzahl von Skelettgelenken, die für eine oder mehrere Aktionen spezifisch sind, die durch den UUO (302) durchgeführt werden, und einem oder mehreren entsprechenden Modellparametern, die für einen normalen Benutzer (NU) (312) vorberechnet werden, wobei der eine oder die mehreren physiologisch interpretierbaren Modellparameter ein oder mehrere angewandte Drehmomente an einem oder mehreren Skelettgelenken der Mehrzahl von Skelettgelenken im Gelenkmodell, Bewegungsgeschwindigkeit an dem einen oder den mehreren Skelettgelenken unter Berücksichtigung und einen oder mehrere Eulerwinkel des einen oder der mehreren Skelettgelenke umfassen; und Detektieren eines oder mehrerer Zustände einer Mehrzahl von neuromuskulären Profilen, wobei die neuromuskulären Profile detektiert werden, wenn Variation(en) in einem oder mehreren Werten, die mit dem einen oder den mehreren abgestimmten physiologisch interpretierbaren Modellparametern assoziiert sind, im Vergleich zu dem einen oder den mehreren entsprechenden Modellparametern, die für den normalen Benutzer (NU) vorberechnet werden, bestimmt werden, und wobei die neuromuskulären Profile modellierte Geschwindigkeitsprofile sind, die eine Darstellung einer zeitlichen Entwicklung von modellierter und beobachteter Geschwindigkeit gegenüber einem Rahmen von Kinect-Daten angeben.

**2.** Prozessorimplementiertes Verfahren nach Anspruch 1, wobei der Schritt des Skalierens eines Gelenkmodells auf der einen oder den mehreren Aktionen basiert, die durch Verfolgen einer realen Bewegung des UUO extrahiert werden.

**3.** System (100), umfassend:

einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

Erhalten von neuromotorischen Daten von einem Kinect®-Sensor, die sich auf einen Benutzer unter Beobachtung (UUO) beziehen, wobei die neuromotorischen Daten Informationen umfassen, die sich auf eine Mehrzahl von Skelettgelenken beziehen, die für eine oder mehrere Aktionen spezifisch sind, die durch den UUO durchgeführt werden,
Skalieren eines Gelenkmodells für jeden UUO durch Identifizieren der einen oder mehreren Aktionen, die an der Mehrzahl von Skelettgelenken durchgeführt werden, um mit einer oder mehreren experimentell erhaltenen Längen der Mehrzahl von Skelettgelenken übereinzustimmen;
Parametrisieren des skalierten Gelenkmodells unter Verwendung eines oder mehrerer physiologisch interpretierbarer Modellparameter, um eine Gelenkkostenfunktion (JC) zu erhalten;
Optimieren der Gelenkkostenfunktion unter Verwendung einer inversen optimalen Steuerungstechnik durch Abstimmen des einen oder der mehreren physiologisch interpretierbaren Modellparameter, wobei die inverse optimale Steuerungstechnik eine Bilevel-Optimierung in der Natur ist und eine optimale Lösung auf zwei Ebenen erforderlich ist, und wobei eine erste Ebene das Finden einer richtigen Trajektorie unter Verwendung einer algebraischen Riccati-Formulierung erfordert und eine zweite Ebene unter Verwendung eines Downhill-Simplex-Algorithmus gelöst wird;
Bestimmen einer Ebene der Abstimmung des einen oder der mehreren physiologisch interpretierbaren Modellparameter basierend auf der optimierten Gelenkkostenfunktion,
Durchführen eines Vergleichs unter Verwendung eines oder mehrerer abgestimmter physiologisch interpretierbarer Modellparameter zwischen der Mehrzahl von Skelettgelenken, die für eine oder mehrere Aktionen spezifisch sind, die durch den UUO (302) durchgeführt werden, und einem oder mehreren entsprechenden Modellparametern, die für einen normalen Benutzer (NU) vorberechnet werden, wobei der eine oder die mehreren physiologisch interpretierbaren Modellparameter ein oder mehrere angewandte Drehmomente an einem oder mehreren Skelettgelenken der Mehrzahl von Skelettgelenken im Gelenkmodell, Bewegungsgeschwindigkeit an dem einen oder den mehreren Skelettgelenken unter Berücksichtigung und einen oder mehrere Eulerwinkel des einen oder der mehreren Skelettgelenke umfassen;
Detektieren eines oder mehrerer Zustände einer Mehrzahl von neuromuskulären Profilen, wobei die neuromuskulären Profile detektiert werden, wenn Variation(en) in einem oder mehreren Werten, die mit dem einen oder den mehreren abgestimmten physiologisch interpretierbaren Modellparametern assoziiert sind, im Vergleich zu dem einen oder den mehreren entsprechenden Modellparametern, die für den normalen Benutzer (NU) vorberechnet werden, bestimmt werden, und wobei die neuromuskulären Profile modellierte Geschwindigkeitsprofile sind, die eine Darstellung einer zeitlichen Entwicklung von modellierter und beobachteter Geschwindigkeit gegenüber einem Rahmen von Kinect-Daten angeben.

**4.** System nach Anspruch 3, wobei das Gelenkmodell basierend auf der einen oder den mehreren Aktionen skaliert wird, die durch Verfolgen einer realen Bewegung des UUO extrahiert werden.

**5.** Ein oder mehrere nichtflüchtige maschinenlesbare Informationsspeichermedien, umfassend eine oder mehrere Anweisungen, die, wenn sie durch einen oder mehrere Hardwareprozessoren ausgeführt werden, bewirken:

Erhalten von neuromotorischen Daten von einem Kinect®-Sensor, die sich auf einen Benutzer unter Beobachtung (UUO) beziehen, wobei die neuromotorischen Daten Informationen umfassen, die sich auf eine Mehrzahl von Skelettgelenken beziehen, die für eine oder mehrere Aktionen spezifisch sind, die durch den UUO durchgeführt werden;

Skalieren eines Gelenkmodells für jeden UUO durch Identifizieren der einen oder mehreren Aktionen, die an der Mehrzahl von Skelettgelenken durchgeführt werden, um mit einer oder mehreren experimentell erhaltenen Längen der Mehrzahl von Skelettgelenken übereinzustimmen;

Parametrisieren des skalierten Gelenkmodells unter Verwendung eines oder mehrerer physiologisch interpretierbarer Modellparameter, um eine Gelenkkostenfunktion (JC) zu erhalten;

Optimieren der Gelenkkostenfunktion unter Verwendung einer inversen optimalen Steuerungstechnik durch Abstimmen des einen oder der mehreren physiologisch interpretierbaren Modellparameter, wobei die inverse optimale Steuerungstechnik eine Bilevel-Optimierung in der Natur ist und eine optimale Lösung auf zwei Ebenen erforderlich ist, und wobei eine erste Ebene das Finden einer richtigen Trajektorie unter Verwendung einer algebraischen Riccati-Formulierung erfordert und eine zweite Ebene unter Verwendung eines Downhill-Simplex-Algorithmus gelöst wird;

Bestimmen einer Ebene der Abstimmung des einen oder der mehreren physiologisch interpretierbaren Modellparameter basierend auf der optimierten Gelenkkostenfunktion;

Durchführen eines Vergleichs unter Verwendung des einen oder der mehreren abgestimmten physiologisch interpretierbaren Modellparameter zwischen der Mehrzahl von Skelettgelenken, die für eine oder mehrere Aktionen spezifisch sind, die durch den UUO (302) durchgeführt werden, und einem oder mehreren entsprechenden Modellparametern, die für einen normalen Benutzer (NU) vorberechnet werden, wobei der eine oder die mehreren physiologisch interpretierbaren Modellparameter ein oder mehrere angewandte Drehmomente an einem oder mehreren Skelettgelenken der Mehrzahl von Skelettgelenken im Gelenkmodell, Bewegungsgeschwindigkeit an dem einen oder den mehreren Skelettgelenken unter Berücksichtigung und einen oder mehrere Eulerwinkel des einen oder der mehreren Skelettgelenke umfassen; und

Detektieren eines oder mehrerer Zustände einer Mehrzahl von neuromuskulären Profilen, wobei die neuromuskulären Profile detektiert werden, wenn Variation(en) in einem oder mehreren Werten, die mit dem einen oder den mehreren abgestimmten physiologisch interpretierbaren Modellparametern assoziiert sind, im Vergleich zu dem einen oder den mehreren entsprechenden Modellparametern, die für den normalen Benutzer (NU) vorberechnet werden, bestimmt werden, und wobei die neuromuskulären Profile modellierte Geschwindigkeitsprofile sind, die eine Darstellung einer zeitlichen Entwicklung von modellierter und beobachteter Geschwindigkeit gegenüber einem Rahmen von Kinect-Daten angeben.

6. Ein oder mehrere nichtflüchtige maschinenlesbare Informationsspeichermedien nach Anspruch 5, wobei der Schritt des Skalierens eines Gelenkmodells auf der einen oder den mehreren Aktionen basiert, die durch Verfolgen einer realen Bewegung des UUO extrahiert werden.

## Revendications

1. Procédé mis en oeuvre par processeur, comprenant :

l'obtention, à partir d'un capteur Kinect®, de données neuromotrices relatives à un utilisateur sous observation (UUO), dans lequel les données neuromotrices comprennent des informations concernant une pluralité d'articulations squelettiques spécifiques à une ou plusieurs actions étant effectuées par l'UUO (302) ;

la mise à l'échelle d'un modèle d'articulation pour chaque UUO par identification des une ou plusieurs actions effectuées sur la pluralité d'articulations squelettiques (304) de façon à correspondre à une ou plusieurs longueurs obtenues de façon expérimentale de la pluralité d'articulations squelettiques ;

le paramétrage du modèle d'articulation mis à l'échelle au moyen d'un ou plusieurs paramètres de modèle physiologiquement interprétables pour obtenir une fonction de coût commun (JC) (306) ;

l'optimisation, au moyen d'une technique de commande optimale inverse, de la fonction de coût commun, par ajustement des un ou plusieurs paramètres de modèle physiologiquement interprétables (308), dans lequel la technique de commande optimale inverse est une optimisation de nature à deux niveaux et une solution optimale est requise à deux niveaux, et dans lequel un premier niveau nécessite de trouver une trajectoire correcte au moyen d'une formulation de Riccati algébrique et un deuxième niveau est résolu au moyen d'un algorithme du simplexe en aval ;

la détermination d'un niveau de l'ajustement des un ou plusieurs paramètres de modèle physiologiquement interprétables sur la base de la fonction de coût commun optimisée (310) ;

la réalisation d'une comparaison au moyen d'un ou plusieurs paramètres de modèle physiologiquement interprétables ajustés entre la pluralité d'articulations squelettiques spécifiques à une ou plusieurs actions étant effectuées par l'UUO (302) et un ou plusieurs paramètres de modèle précalculés correspondants pour un utilisateur normal (NU) (312), dans lequel les un ou plusieurs paramètres de modèle physiologiquement interprétables comprennent un ou plusieurs couples appliqués à une ou plusieurs articulations squelettiques de la pluralité d'articulations squelettiques dans le modèle d'articulation, la vitesse de mouvement au niveau des une ou plusieurs articulations squelettiques à l'étude et un ou plusieurs angles d'Euler des une ou plusieurs articulations squelettiques ; et

la détection d'un ou plusieurs états d'une pluralité de profils neuromusculaires, dans lequel les profils neuromusculaires sont détectés lorsque des variations d'une ou plusieurs valeurs associées aux un ou plusieurs paramètres de modèle physiologiquement interprétables ajustés sont déterminées par comparaison aux un ou plusieurs paramètres de modèle précalculés correspondants pour l'utilisateur normal (NU), et dans lequel les profils neuromusculaires sont des profils de vitesse modélisés qui indiquent un tracé d'une évolution temporelle de la vitesse modélisée et observée vs. une trame de données Kinect.

2. Procédé mis en oeuvre par processeur selon la revendication 1, dans lequel l'étape de mise à l'échelle d'un modèle d'articulation est basée sur les une ou plusieurs actions qui sont extraites par le suivi des mouvements dans le monde réel de l'UUO.

3. Système (100), comprenant :

une mémoire (102) stockant des instructions ;
une ou plusieurs interfaces de communication (106) ; et
un ou plusieurs processeurs matériels (104) couplés à la mémoire (102) par l'intermédiaire des une ou plusieurs interfaces de communication (106), dans lequel les un ou plusieurs processeurs matériels (104) sont configurés par les instructions pour :

obtenir, à partir d'un capteur Kinect®, des données neuromotrices relatives à un utilisateur sous observation (UUO), dans lequel les données neuromotrices comprennent des informations concernant une pluralité d'articulations squelettiques spécifiques à une ou plusieurs actions étant effectuées par l'UUO ;

mettre à l'échelle un modèle d'articulation pour chaque UUO par identification des une ou plusieurs actions effectuées sur la pluralité d'articulations squelettiques de façon à correspondre à une ou plusieurs longueurs obtenues de façon expérimentale de la pluralité d'articulations squelettiques ;

paramétrer le modèle d'articulation mis à l'échelle au moyen d'un ou plusieurs paramètres de modèle physiologiquement interprétables pour obtenir une fonction de coût commun (JC) ;

optimiser, au moyen d'une technique de commande optimale inverse, la fonction de coût commun, par ajustement des un ou plusieurs paramètres de modèle physiologiquement interprétables, dans lequel la technique de commande optimale inverse est une optimisation de nature à deux niveaux et une solution optimale est requise à deux niveaux, et dans lequel un premier niveau nécessite de trouver une trajectoire correcte au moyen d'une formulation de Riccati algébrique et un deuxième niveau est résolu au moyen d'un algorithme du simplexe en aval ;

déterminer un niveau de l'ajustement des un ou plusieurs paramètres de modèle physiologiquement interprétables sur la base de la fonction de coût commun optimisée ;

réaliser une comparaison au moyen d'un ou plusieurs paramètres de modèle physiologiquement interprétables ajustés entre la pluralité d'articulations squelettiques spécifiques à une ou plusieurs actions étant effectuées par l'UUO (302) et un ou plusieurs paramètres de modèle précalculés correspondants pour un utilisateur normal (NU), dans lequel les un ou plusieurs paramètres de modèle physiologiquement interprétables comprennent un ou plusieurs couples appliqués à une ou plusieurs articulations squelettiques de la pluralité d'articulations squelettiques dans le modèle d'articulation, la vitesse de mouvement au niveau des une ou plusieurs articulations squelettiques à l'étude et un ou plusieurs angles d'Euler des une ou plusieurs articulations squelettiques ;

détecter un ou plusieurs états d'une pluralité de profils neuromusculaires, dans lequel les profils neuromusculaires sont détectés lorsque des variations d'une ou plusieurs valeurs associées aux un ou plusieurs paramètres de modèle physiologiquement interprétables ajustés sont déterminées par comparaison aux un ou plusieurs paramètres de modèle précalculés correspondants pour l'utilisateur normal (NU), et dans lequel les profils neuromusculaires sont des profils de vitesse modélisés qui indiquent un tracé d'une évolution temporelle de la vitesse modélisée et observée vs. une trame de données Kinect.

4. Système selon la revendication 3, dans lequel le modèle d'articulation est mis à l'échelle sur la base des une ou plusieurs actions qui sont extraites par le suivi des mouvements dans le monde réel de l'UUO.

5. Un ou plusieurs supports de stockage d'informations lisibles par machine non transitoires comprenant une ou plusieurs instructions qui, lorsqu'elles sont exécutées par un ou plusieurs processeurs matériels entraînent :

l'obtention, à partir d'un capteur Kinect®, de données neuromotrices relatives à un utilisateur sous observation (UUO), dans lesquels les données neuromotrices comprennent des informations concernant une pluralité d'articulations squelettiques spécifiques à une ou plusieurs actions étant effectuées par l'UUO ;
la mise à l'échelle d'un modèle d'articulation pour chaque UUO par identification des une ou plusieurs actions effectuées sur la pluralité d'articulations squelettiques de façon à correspondre à une ou plusieurs longueurs obtenues de façon expérimentale de la pluralité d'articulations squelettiques ;
le paramétrage du modèle d'articulation mis à l'échelle au moyen d'un ou plusieurs paramètres de modèle physiologiquement interprétables pour obtenir une fonction de coût commun (JC) ;
l'optimisation, au moyen d'une technique de commande optimale inverse, de la fonction de coût commun, par ajustement des un ou plusieurs paramètres de modèle physiologiquement interprétables, dans lesquels la technique de commande optimale inverse est une optimisation de nature à deux niveaux et une solution optimale est requise à deux niveaux, et dans lesquels un premier niveau nécessite de trouver une trajectoire correcte au moyen d'une formulation de Riccati algébrique et un deuxième niveau est résolu au moyen d'un algorithme du simplexe en aval ;
la détermination d'un niveau de l'ajustement des un ou plusieurs paramètres de modèle physiologiquement interprétables sur la base de la fonction de coût commun optimisée ;
la réalisation d'une comparaison au moyen d'un ou plusieurs paramètres de modèle physiologiquement interprétables ajustés entre la pluralité d'articulations squelettiques spécifiques à une ou plusieurs actions étant effectuées par l'UUO (302) et un ou plusieurs paramètres de modèle précalculés correspondants pour un utilisateur normal (NU), dans lesquels les un ou plusieurs paramètres de modèle physiologiquement interprétables comprennent un ou plusieurs couples appliqués à une ou plusieurs articulations squelettiques de la pluralité d'articulations squelettiques dans le modèle d'articulation, la vitesse de mouvement au niveau des une ou plusieurs articulations squelettiques à l'étude et un ou plusieurs angles d'Euler des une ou plusieurs articulations squelettiques ; et
la détection d'un ou plusieurs états d'une pluralité de profils neuromusculaires, dans lesquels les profils neuromusculaires sont détectés lorsque des variations d'une ou plusieurs valeurs associées aux un ou plusieurs paramètres de modèle physiologiquement interprétables ajustés sont déterminées par comparaison aux un ou plusieurs paramètres de modèle précalculés correspondants pour l'utilisateur normal (NU), et dans lesquels les profils neuromusculaires sont des profils de vitesse modélisés qui indiquent un tracé d'une évolution temporelle de la vitesse modélisée et observée vs. une trame de données Kinect.

6. Un ou plusieurs supports de stockage d'informations lisibles par machine non transitoires selon la revendication 5, dans lesquels l'étape de mise à l'échelle d'un modèle d'articulation est basée sur les une ou plusieurs actions qui sont extraites par le suivi des mouvements dans le monde réel de l'UUO.

SYSTEM
100

MEMORY
102

108

HARDWARE
PROCESSOR(S)
104

INTERFACE(S)
106

FIG. 1

FIG. 2

Obtaining from a Kinect® sensor, neuro-motor data pertaining to a user under observation (UUO), wherein the neuro motor data comprises information pertaining to a plurality of skeletal joints specific to one or more actions being performed by the UUO  ⌐⌐ 302

↓

Scaling a joint model by identifying the one or more actions performed on the plurality of skeletal joints  ⌐⌐ 304

↓

Parameterizing the scaled joint model using one or more physiologically interpretable model parameters to obtain a joint cost function (JC)  ⌐⌐ 306

↓

Optimizing, using an inverse optimal control technique, the joint cost function by tuning the one or more physiologically interpretable model parameters  ⌐⌐ 308

↓

Determining a level of the tuning of the one or more physiologically interpretable model parameters based on the optimized joint cost function  ⌐⌐ 310

↓

Performing based on the level of the tuning, a comparison between the one or more tuned physiologically interpretable model parameters with one or more corresponding model parameters pre-computed for a normal user (NU)  ⌐⌐ 312

↓

Detecting one or more neuro muscular profiles based on the comparison  ⌐⌐ 314

**FIG. 3**

FIG. 4B

FIG. 4A

FIG. 4D

FIG. 4C

**FIG. 5**

FIG. 6A

FIG. 6B

FIG. 6C

FIG. 7A

FIG. 7B

FIG. 8B

FIG. 8A

**REFERENCES CITED IN THE DESCRIPTION**

**Patent documents cited in the description**

- US 2017287146 A

- US 2011054870 A